Europäisches Patentamt

European Patent Office

Office européen des brevets

(11) Numéro de publication: **0 067 726 B1**

## (12) FASCICULE DE BREVET EUROPEEN

(45) Date de publication du fascicule du brevet:
16.01.85

(21) Numéro de dépôt: 82400812.2

(22) Date de dépôt: 04.05.82

(51) Int. Cl.⁴: **A 61 B 5/14**

---

(54) **Instrument de microprélèvement du sang.**

---

(30) Priorité: 04.06.81 FR 8111074

(43) Date de publication de la demande:
22.12.82 Bulletin 82/51

(45) Mention de la délivrance du brevet:
16.01.85 Bulletin 85/3

(84) Etats contractants désignés:
AT BE CH DE GB IT LI LU NL SE

(56) Documents cités:
FR - A - 1 133 137
FR - A - 2 076 366
FR - A - 2 281 740
US - A - 2 702 549
US - A - 3 741 197

(73) Titulaire: Labarthe, Jean-Christophe, 10, rue du Petit
Saint-Martin, F-37000 Tours (FR)

(72) Inventeur: Labarthe, Jean-Christophe, 10, rue du Petit
Saint-Martin, F-37000 Tours (FR)

(74) Mandataire: Flechner, Willy et al, CABINET
FLECHNER 22, Avenue de Friedland, F-75008 Paris (FR)

---

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention se rapporte aux instruments de microprélèvement du sang et, plus particulièrement, aux pipettes de prélèvement pour effectuer des microhémocultures.

La microhémoculture consiste à déterminer la présence ou l'absence de germes dans le sang en en mettant de très faibles quantités sur un milieu de culture convenable. Le volume de sang nécessaire est souvent de l'ordre de 200 μl seulement, ce qui permet de le prélever en piquant simplement la peau à l'aide d'une lancette, puis en recueillant le sang par capillarité dans une pipette, dite pipette de Pasteur, munie d'un tube effilé, en sorte que le sang est immédiatement à l'abri de toute pollution par l'atmosphère environnante. Cette technique s'impose chaque fois qu'une veine est difficile à localiser, notamment chez les nouveau-nés, mais aussi chez les jeunes enfants et chez les malades dont le patrimoine veineux est insuffisant.

Cette technique, dont la demande de brevet FR-A No 2281740 fournit un exposé, n'est pas sans inconvénient. Entre l'instant où l'on pique la peau au talon d'un nouveau-né et celui où l'on applique la pipette sur le sang qui perle, il s'écoule un temps suffisant pour que le nouveau-né s'agite et pour que la goutte de sang vienne en contact avec une couche polluée de selles ou autres ou s'étale sur la peau du talon. Le sang se charge ainsi de germes, dits souillures, externes au système sanguin et qui, détectés lors de la microhémoculture, portent à croire, à tort, que le nouveau-né est atteint d'une maladie.

L'invention pallie cet inconvénient, en diminuant beaucoup les dangers de souillure du micro-échantillon de sang lors du prélèvement, à l'aide d'un instrument simple, rapide à manipuler et à fabriquer, et qui peut être du type à jeter après usage.

L'invention a donc pour objet un instrument de microprélèvement du sang qui comprend un corps tubulaire qui se continue en un tube capillaire et duquel part une barrette terminée par une pointe.

Pour effectuer le prélèvement, on pique d'abord la peau à l'aide de la pointe de la barrette puis, en déplaçant simplement l'instrument, on amène le tube capillaire sur l'endroit piqué, avant que le sang n'ait pu s'étaler et sans lâcher le talon du nouveau-né où l'on a effectué la piqûre pour lâcher une lancette et se saisir d'une pipette comme c'était le cas jusqu'ici.

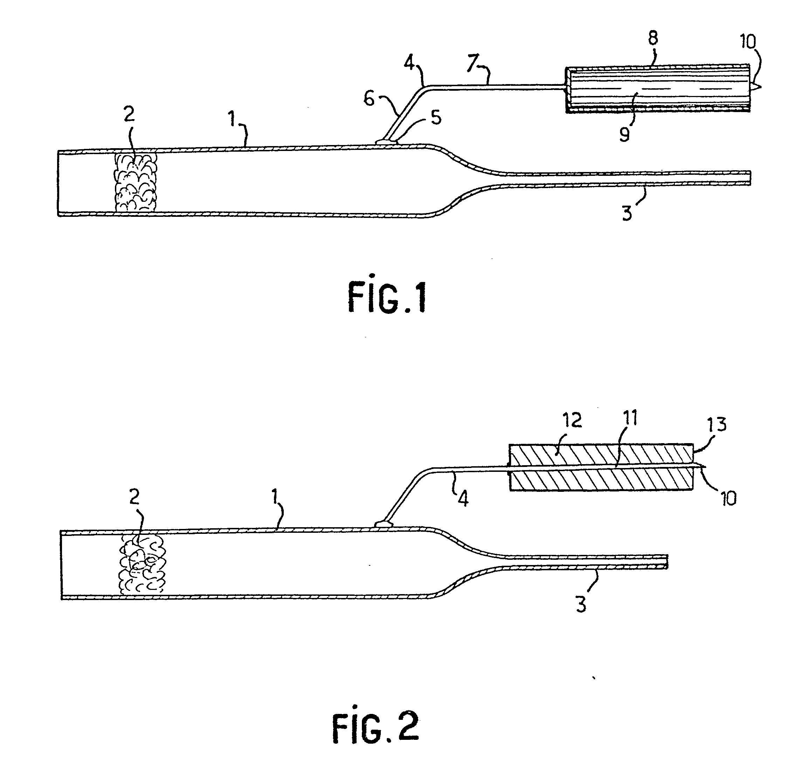

Au dessin annexé, donné uniquement à titre d'exemple, les fig. 1 et 2 sont des vues en coupe de deux variantes d'instrument de microprélèvement suivant l'invention.

L'instrument de la fig. 1 comprend un corps 1 tubulaire, en verre, ouvert à l'une des extrémités mais bouché par un tampon 2 de coton qui y est engagé. Le corps 1 tubulaire se continue en un tube 3 capillaire. L'ensemble du corps 1 et du tube 3 constitue une pipette de Pasteur classique.

Du corps 1 part une barrette 4 en verre plein. La barrette 4 est soudée en 5 à la paroi extérieure du corps 1. La barrette comprend un tronçon 6, qui part de l'endroit 5 de soudure et qui est suivi, suivant un coude, d'un tronçon 7 sensiblement parallèle au tube 3 et allant dans le même sens que celui-ci à partir du corps 1.

A l'extrémité de la barrette 4, celle-ci s'épanouit en un épanouissement 8 tubulaire de réception d'une lancette 9 ou vaccinostyle à pointe 10. Le corps de lancette 9 a la même longueur que l'épanouissement 8. La pointe 10 et l'extrémité du tube 3 sont sensiblement dans le même plan.

A la fig. 2, le corps 1 forme aussi sensiblement la hampe d'un Y, dont les deux barres supérieures sont formées par le tube 3 capillaire et par la barrette 4, mais la pointe 10 est plus éloignée du corps 1 que ne l'est l'extrémité du tube 3 capillaire. On ne court pas le danger de frotter involontairement la peau de l'extrémité libre du tube 3 capillaire et de le polluer, quand on pique la pointe 10 dans la peau.

La pointe 10 est à l'extrémité d'une tige 11, en métal, soudée à la barrette 4 en verre et entourée d'un manchon 12, dont la face 13 frontale forme garde pour la pointe 10.

La distance entre la pointe 10 et l'extrémité libre du tube 3 capillaire est suffisante cependant pour que, lors du prélèvement, la pointe 10 se trouve hors du contour du corps.

On peut réaliser le tube capillaire en verre. On peut aussi conférer de la capillarité à une matière plastique, telle que le polystyrène, en y appliquant une substance convenable, par exemple en la trempant dans un bain de Sunhaptol-O (fourni par PCUK France) qui est un agent tensio-actif non toxique et sans action chimique sur la matière plastique, dérivé par exemple d'un alcool gras condensé avec 5 à 15 mol d'oxyde d'éthylène, notamment d'alcool laurylique condensé avec 5 à 15 mol d'oxyde d'éthylène, notamment d'alcool laurylique condensé avec 9 mol d'oxyde d'éthylène.

Les analyses suivantes illustrent l'invention.

*Analyse 1 :*

On effectue des microhémocultures en utilisant la technique classique de prélèvement, avec pipette et lancette séparées, et des macrohémocultures par prélèvement veineux chez 411 sujets donc 293 nouveau-nés (1 à 30 d) et 27 jeunes enfants (moyenne de 6 ans). Il y a 367 résultats négatifs dans les deux cas. Il y a 16 résultats positifs pour l'un des deux types d'hémoculture ou pour les deux à la fois et il y a 18 résultats dits de souillure pour lesquels l'une au moins des hémocultures révèle un germe alors qu'il est absent d'hémocultures suivantes de contrôle et que le sujet ne présente pas de signe clinique d'infection. Les souillures se répartissent de la manière suivante:

| Macro-hémo-cultures | Micro-hémo-cultures | Micro- et macrohémo-cultures | Total |
|---|---|---|---|
| 3 (0,7%) | 15 (3,6%) | 0 | 18 |

→

La microhémoculture donne des résultats erronés dans 3,6% des cas.

*Analyse 2:*

On reprend l'analyse 1 sur 200 enfants, mais en utilisant la pipette-lancette d'un seul tenant suivant l'invention. Il y a 8 résultats positifs et 5 résultats dits de souillure qui se répartissent comme suit:

| Macro-hémo-cultures | Micro-hémo-cultures | Micro- et macrohémo-cultures | Total |
|---|---|---|---|
| 2 (1%) | 3 (1,5%) | 0 | 5 |

Les résultats erronés de la microhémoculture ne représentent plus que 1,5% des cas, ce qui est comparable à ce que donne la macrohémoculture, avec cependant les avantages de facilité de prélèvement propre à la microhémoculture.

**Revendications**

1. Instrument de microprélèvement du sang comprenant un corps tubulaire, qui se continue en un tube capillaire, caractérisé en ce que du corps part une barrette terminée par une pointe.

2. Instrument selon la revendication 1, caractérisé en ce que la pointe et l'extrémité du tube capillaire sont sensiblement dans un même plan.

3. Instrument selon la revendication 1, caractérisé en ce que la pointe est plus éloignée du corps que ne l'est l'extrémité du tube capillaire.

4. Instrument selon l'une des revendications 1, 2 ou 3, caractérisé en ce que la barrette comprend un épanouissement tubulaire de réception d'un vaccinostyle formant la pointe.

5. Instrument selon l'une des revendications 1 à 4, caractérisé en ce que le corps forme sensiblement la hampe d'un Y dont les deux barres supérieures sont formées par la barrette et par le tube capillaire.

**Patentansprüche**

1. Instrument zur Mikroentnahme einer Blutprobe, bestehend aus einem röhrenförmigen Körper, der in einer Kapillare ausläuft, dadurch gekennzeichnet, dass vom Körper ein in einer Spitze auslaufender Nadelstab ausgeht.

2. Instrument nach Anspruch 1, dadurch gekennzeichnet, dass die Spitze des Nadelstabes und das Ende der Kapillare des Körpers im wesentlichen in der gleichen Ebene liegen.

3. Instrument nach Anspruch 1, dadurch gekennzeichnet, dass die Spitze des Nadelstabes über das Ende der Kapillare des röhrenförmigen Körpers vorsteht.

4. Instrument nach Anspruch 1, 2 oder 3, dadurch gekennzeichnet, dass der Nadelstab eine röhrenförmige Erweiterung zur Aufnahme einer die Spitze bildenden Impfnadel aufweist.

5. Instrument nach einem der Ansprüche 1 bis 4, dadurch gekennzeichnet, dass der Körper im wesentlichen den Stiel eines Y bildet und die beiden oberen Balken des Y durch den Nadelstab und durch die Kapillare des Körpers gebildet sind.

**Claims**

1. Instrument for taking blood microsamples, comprising a tubular body extending into a capillary tube, and a rod which terminates in a point and which branches from the body.

2. Instrument as set forth in Claim 1, wherein the point and the end of the capillary tube are substantially in the same plane.

3. Instrument as set forth in Claim 1, wherein the point is further from the body than is the end of the capillary tube.

4. Instrument as set forth in Claim 1, 2 or 3, wherein the rod comprises a widened tubular reception portion for a vaccination needle forming the point.

5. Instrument as set forth in Claims 1-4, wherein the body basically forms the upright of a Y whose two upper arms are formed by the rod and by the capillary tube.

FIG.1

FIG.2